(19) Europäisches Patentamt
European Patent Office
Office européen des brevets

(11) **EP 1 569 631 B1**

(12) **EUROPEAN PATENT SPECIFICATION**

(45) Date of publication and mention
of the grant of the patent:
**29.08.2007 Bulletin 2007/35**

(51) Int Cl.:
*A61K 31/121* (2006.01)    *A61P 11/06* (2006.01)

(21) Application number: **02783467.0**

(22) Date of filing: **02.12.2002**

(86) International application number:
**PCT/IB2002/005065**

(87) International publication number:
**WO 2004/050071 (17.06.2004 Gazette 2004/25)**

(54) **A METHOD OF PREVENTING AND/OR TREATING ASTHMA USING PARABROMOPHENACYL BROMIDE (PBPB)**

VERFAHREN ZUR PRÄVENTION UND/ODER BEHANDLUNG VON ASTHMA MIT PARABROMOPHENACYL BROMIDE (PBPB)

METHODE DE PREVENTION ET/OU TRAITEMENT DE L'ASTHME UTILISANT DU BROMURE DE PARABROMOPHENACYLE (PBPB)

(84) Designated Contracting States:
**AT BE BG CH CY CZ DE DK EE ES FI FR GB GR IE IT LI LU MC NL PT SE SI SK TR**

(43) Date of publication of application:
**07.09.2005 Bulletin 2005/36**

(73) Proprietor: **COUNCIL OF SCIENTIFIC AND INDUSTRIAL RESEARCH**
**New Delhi 100 001 (IN)**

(72) Inventors:
• **RAM, Arjun**
  **Delhi 110 007 (IN)**
• **GHOSH, Balaram**
  **Delhi 110 007 (IN)**
• **GANGAL, Sharad Vishwanath**
  **Thane 400 602 (IN)**

(74) Representative: **Gaunt, Robert John**
**Stevens Hewlett & Perkins**
**Halton House**
**20/23 Holborn**
**GB-London EC1N 2JD (GB)**

(56) References cited:
WO-A-96/40982          US-A- 4 933 365
US-A- 5 328 842        US-A- 5 354 677
US-A- 5 948 626

• WHITE STEVEN R ET AL: "Regulation of human eosinophil degranulation and activation by endogenous phospholipase A-2." JOURNAL OF CLINICAL INVESTIGATION, vol. 91, no. 5, 1993, pages 2118-2125, XP009012187 ISSN: 0021-9738
• BALSINDE JESUS ET AL: "Regulation and inhibition of phospholipase A2." ANNUAL REVIEW OF PHARMACOLOGY AND TOXICOLOGY, vol. 39, 1999, pages 175-189, XP001152698 1999 Annual Reviews Inc. P.O. Box 10139, 4139 El Camino Way, Palo Alto, California 94306, USA ISBN: 0-8243-0439-X
• G: S. VON ROSSUM: "Regulation of cytosolic PLA2 activity plays a central role in cell responses" PHD THESIS, [Online] 24 September 2001 (2001-09-24), XP002244301 Retrieved from the Internet: <URL:http://www.library.uu.nl/ digiarchief/ dip/diss/ 2002-1203-083248/inhoud.htm> [retrieved on 2003-06-13]

## Description

### Technical field

The present invention relates to the use of parabromophenacyl bromide (PBPB) for the manufacture of a medicament for preventing and/or treating asthma. It further relates to the use of PBPB for the manufacture of a medicament for modulating levels of biomolecules to achieve the same.

### Background Art

Asthma is an inflammatory disorder of airways, which is characterized by reversible airway obstruction, airway hyper reactivity, high serum IgE and eosinophils levels in the airways. This disease affects millions of people world wide and reaching epidemic proportions (Cookson, 1999).

With the increasing understanding of the molecular mechanism of asthma, many therapeutic options such as glucocorticoids, mediators antagonists, cytokine modulators, phosphodiesterase-4 inhibitors, chromone compounds, and immunotherapy have been reported (Barnes, 1999). However, steroids are still a mainstay for the management of asthma. Steroids therapy has many side effects such as osteoporosis, obesity, impaired wound healing, increased risk of infection, suppression of hypothamic-pituitary-adrenal axis, myopathy, hypertension etc. (stites et al., 1997) and there is no escape from its adverse side effects. Therefore, there exists a need of search for non-steroidal anti-asthmatic agents with very low or negligible side effects.

Phospholipase $A_2$ (PL $A_2$) is a key enzyme in generating various arachidonic acid metabolites, such as leukotrines and prostasglandins, which are involved pathogenesis of various inflammatory diseases including asthma (bowton et al 1997, chilton et al 1996, drazen et al. 1999). PBPB has been found to block edema and myotoxicity (melo and ownby, 1999; evans and ownby, 1993).

Recently, it has been demonstrated to inhibit several other steps involved in the manifestation of inflammation. Tithof and coworkers demonstrated that PBPB inhibits polyvinylchloride-induced neutrophils superoxide (02) release (Tithof et aL, 1996). Further, this compound is reported to inhibit the binding activity of NF-kB (von puijenbrock et al., 1999), an important transcription factor involved in the expression of various inflammatory cytokines, to DNA. PBPB has also been demonstrated to reduce the adhesion of certain bacterial cells to the endothelial cells in guinea pig colon (Guhathakurta et al, 1999), an *in-vitro* study by Detsouli and coworkers (1985) demonstrated that PBPB reduce the construction of lung parenchymal strips induced by platelet activating factor (PAF) or ovalbumin. Many of these parameters, i.e., increase of pl a2 activity (Bowton et al., 1997; Mehta et al., 1990), binding of NF-kb to DNA (Barnes., 1996), superoxide release (Barnes., 1990) and cell adhesion (Gundel et al., 1991) are involved in the pathogenesis of asthma. However, till date there is no direct *in-vivo* experiment report that can demonstrate the effect of PBPB on asthmatic features either in human or animal model. Novelty of the invention is in first *in-vivo* demonstration of PBPB for alleviation the characteristic features of asthma produced in mouse such as allergen-induced early airway response (ear) and late airway response (LAR).

### Objects of the present invention

The main object of the present invention is to develop a medicament for preventing and/or treating asthma.

Another object of the present invention is to develop a medicament for preventing and/or treating asthma in animals including humans, using PBPB.

Yet another object of the present invention is to determine the dosage schedule of PBPB for preventing and/or treating asthma.

Still mother object of the present invention is to determine the routes for administration of PBPB for preventing and/or treating asthma in animals.

Still another object of the present invention is to develop a medicament for modulating levels of biomolecules to help prevent and/or treat asthma.

Still another object of the present invention is to determine the effect of PBPB on IFN-gamma levels.

Still another object of the present invention is to determine the effect of PBPB on IL-4, IL-5 and IL-13 levels.

Still another object of the present invention is to determine the effect of PBPB on eosinophils levels.

Still another object of the present invention is to determine the effect of PBPB on IgE levels.

Still another object of the present invention is to determine the effect of PBPB on airway constriction (SGaw).

Still another object of the present invention is to determine the effect of PBPB on airway reactivity.

Another main object of the present invention is to provide a lead molecule for the prevention of development of asthma symptoms.

Yet another object of the invention is to provide a lead molecule for development of a therapeutic agent, which can alleviate the characteristic asthmatic features.

## Summary to the present invention

The present invention relates to the use of parabromophenacyl bromide (PBPB) for the manufacture of a medicament for preventing and/or treating asthma. It further relates to the user of PBPB for the manufacture of a medicament for modulating levels of biomolecules to achieve the same.

## Detailed description of the present invention

Accordingly, the present invention relates to a medicament for preventing and/or treating asthma in a subject, said medicament comprising an effective pharmacological amount of parabromophenacyl bromide (PBPB). It further relates to such a medicament for modulating levels of biomolecules to achieve the same. The medicament is for administration to an animal or a human.

Typically, the concentration of PBPB is ranging between 0.1 to 10 mg/kg body weight. Preferably, the concentration of PBPB is about 1 mg/kg body weight. Typically, the medicament containing the PBPB is administered for about one week. It is considered that the PBPB is free of side effects.

The medicament of the present invention may be is administered through either of the intra-peritoneum and the oral routes. In another embodiment of the present invention, there is provided a medicament containing PBPB for modulating levels of biomolecules to help prevent and/or treat asthma. Variation in the levels of biomolecules is measured after its administration. In one aspect of the present invention, the PBPB helps maintain IFN-gamma levels.

In another aspect of the present invention, the PBPB helps attenuate IL-4, IL-5 and IL-13 levels.

In still another aspect of the present invention, the PBPB prevents an increase in IL-4, IL-5 and IL-13 levels.

In still another aspect of the present invention, the PBPB helps attenuate eosinophils levels.

In still another aspect of the present invention, the PBPB prevents an increase in eosinophils levels.

In still another aspect of the present invention, the PBPB helps attenuate IGE levels by about 50%.

In still another aspect of the present invention, the PBPB prevents an increase in IGE levels.

In still another aspect of the present invention, the PBPB helps prevent airway constriction (SGaw).

In still another aspect of the present invention, the PBPB helps recover up to about 96% of basal level airway constriction (SGaw).

In still another aspect of the present invention, the PBPB helps prevent airway reactivity.

In still another aspect of the present invention, the PBPB helps prevent airway reactivity.

## Brief description of the accompanying drawings:

**Figure 1** shows protocol for both preventive and curative effect of PBPB.
**Figure 2** shows measurement of airway calibre using mouse as a model system.
**Figure 3** shows variation in levels of specific airway conductance before and after ova aerosol challenge in both control and PBPB treated mice.
**Figure 4** shows reversal of SGaw, reflecting curative effect of PBPB in ova aerosol challenged mice.
**Figure 5** shows variation in levels of specific airway conductance before and after methacholine (MCh) challenge in both control and PBPB treated mice.
**Figure 6** shows reversal of SGaw, reflecting curative effect of PBPB in methacholine (MCh) challenged mice.
**Figure 7** shows reducing effect of PBPB in serum IgE levels,
**Figure 8** shows inhibitory effect of PBPB in eosinophils in BAL fluid.

The existing anti-asthmatic drugs, particularly steroids, have many side effects. There is intense need to develop certain non-steroidal anti-asthmatic drugs. In this context, para-bromophenacyl bromide (PBPB), a non-steroidal anti-inflammatory compound, was tested for anti-asthmatic activity using a mouse model of asthma. Administration of a pharmacologically effective dose of PBPB to mice during sensitization prevented the development of both the asthmatic features (ear and lar). This finding showed a preventive effect of PBPB on the development of asthma. The present invention also showed that PBPB, when administered orally to the animals already showing impaired airways features, alleviated the existing impaired features. PBPB has been found to retain IFN-y levels and attenuate IL-4, IL-5 and eosinophils levels in the bronchoalveolar lavage (bal) fluid. The allergen-specific IgE levels in the sera samples were also reduced significantly.

The present invention is based on the novel use of para-bromophenacyl bromide as an anti-asthmatic agent.

The method of testing comprises of:

> a sensitizing the animals by an anti-IgEnic protein to induce characteristic asthmatic features,
> b. estimating asthmatic features prior to, during and after sensitization of the animals,
> c. administering pharmacologically active concentration of a solution of PBPB to healthy animals during and after sensitization and
> d measuring immunological features in the sacrificed animals after step (b) and (c). The animal model used may be selected from balb/c mice, rabbits and guinea pigs.

The protein for sensitizing the animals may be administered through intraperitoneally injection or aerosol inhalation routes.

The protein solution in normal saline used for sensitization may be selected from ovalbumin, bovine serum albumin or any other antIgEnic protein, in a concentration ranging from 10-100 ng per injection or 1-5% for inhalation by aerosol in normal saline.

PBPB may be administered orally to the animals in the concentration range of 0.1 to 10 mg/kg body weight.

The asthmatic features may be estimated by known methods of measuring specific airway conductance or specific airway resistance.

The immunological features may be measured by estimating IgE, IFN-gamma, IL-4, IL-5 and eosinophils levels by known methods. Asthma is an inflammatory disease of the airways which affects millions of people worldwide. The disease is reaching epidemic proportions [Cookson, 1999] and young lives are increasingly rendered unproductive. Asthma is characterized by airway obstruction, airway hyper reactivity, and high IgE levels in the serum acid eosinophils in the airways. The development of this disease is mediated by proinflammatory cytokines-IL-4, IL-13 and IL-5 secreted by th2 cells. The cytokine, interferon-gamma (IFN-y) secreted by the th1 cells, on the other hand, inhibits the: th2 cytokines. The asthmatic response may be divided into early-and late phase reactions. The early phase begins immediately after secondary exposure to the allergen and is mediated by histamine and other lipid mediators, which result in inflammation and airway constriction. The late phase reaction occurs 8-24 hours after and results in inflammation of inflammatory cells, e.g., eosinophils, neutrophils etc. in The alveoli. These cells release toxic granule proteins, which damage the epithelium, and also produce a variety of mediators including lipid response may be divided into early-and late phase reactions. The early phase begins immediately after secondary exposure to the allergen and is mediated by histamine and other lipid mediators, which result in inflammation and airway constriction. The late phase reaction occurs 8-24 hours after and results in infiltration of inflammatory cells, e.g., eosinophils, neutrophils etc in the alveoli. These cells release toxic granule proteins, which damage the epithelium and also produce a variety of mediators including lipid mediators [Barnes et al., 1998].

With the increasing understanding of the molecular mechanism of asthma, many therapeutic options have been reported (Barnes, 1999). However, after over 40 years use of steroids, there is no substitute so far and steroids are still a major drug along with beta2-adrenoceptor agonists for the management of asthma. There is no escape from its adverse side effects. Therefore, there is a need to search for non-steroidal anti-asthmatk agents with very low or negligible side effects.

In this context, PBPB was tested on a mouse model of asthma. Mice were sensitized with intraperitoneally and aerosol inhalation of ova to develop the characteristic features of asthma such as allergen induced early airway response (ear) and late airway response (lar). These asthmatic features were characterized by measuring airway caliber in the term of specific airway conductance (SGaw) by a non-invasive technique, dual-chamber whole body plethysmography. After developing the characteristic features (ear and lar) m mouse, the compound. PBPB was given orally during the whole sensitization period to test the preventive effect on the development of asthmatic features. To examine the therapeutic effect of PBPB on the asthmatic features, it was fed for one week to mice after sensitization and confirming their asthmatic features.

After testing the compound for the preventive as well as for therapeutic values in intact conscious mice, mice were sacrificed for Collecting the blood and bronchoalveolar lavage (bal) fluid to measure the levels of IgE, cytokines 11-4, IL,5 and IFN-y and homophile Ovalbumin specific IgE levels in the sera and the levels of IL-4, IL-5 and IFN-y in the BAL fluid were measured by Elisa kits. The prevalence of eosinophils was determined in BAL fluid by flow cytometry.

The present invention provides an effective compound for preventing the development of the characteristic features of asthma in an animal. For example, there was prevention of the development of airway constriction and airway reactivity in mice treated orally with PBPB durings sensitization.

The present invention also demonstrates that PBPB is effective when given to mice after sensitization, i.e., after developing airway hyper reactivity. PBPB administered orally fur one week to airway hyper reactive animals, inhibited both allergen induced airway constriction and army hyper reactivity to methacoline. This showed the therapeutic potential of this compound.

The present invention also shows that PBPB reduces sensitization as reduced serum IgE levels indicate it;

and retained IFN-y levels in the BAL fluid.

PBPB administration to the mice both during sensitization as wall as after sensitization reduced significantly the prevalence of eosinophils in the BAL fluid. This finding suggests that PBPB inhibits inflammation by administering its pharmacologically effective dose. The effective dose was found to be 1 mg/kg body weight.

The present invention provides an effective agent, para-bromophenacyl bromide (PBPB), which prevents the development of characteristic features of asthma such as allergen-induced early airway response (ear) and late airway response (lar) in an animal model by administering a pharmacologically effective dose to the said animal the invention also provides that PBPB can attenuate already developed ear and Jar in the said animal giving its pharmacologically effective dose after sensitization. This showed the therapeutic effect of this compound. The present invention also demonstrates that that PBPB attenuates the alteration of certain immunological parameters, viz, eosinophils, lgE, IL-4, IL-5 and IFN-y, associated with asthma. Thus, PBPB, being a non-steroidal compound, can be used as a lead molecule in the development of anti-asthmatic drugs. The present invention relates to novel use of para-bromophenacyl bromide as an asthmatic agent Para-bromophenacyl bromide (PBPB) has been shown here to have potential as a lead molecule for the development of anti-asthmatic drug for the treatment of clinical asthma.

The following examples are given for the purpose of illustrating various embodiments of the invention.

### Example 1- Animals sensitization:

BALB/c male mice, eight-ten weeks old, weighing 18-22 grams were acclimatized for one week under the laboratory conditions. Mice were sensitized by injecting intraperitoneally 02 ml of saline containing 10 micrograms ovalbumin (ova) (Sigma, USA) adsorbed on 2 mg aluminum hydroxide gel on days 0, 7 and 14 followed by aerosol inhalation for 5 consecutive days, from day 19 to 23 with 2% ova (in saline w/v) for 30 minutes daily. Aerosol challenge was performed by placing mice in Plexiglas chamber (20 x 20 x 10 cm$^3$) and ova or saline alone was aerosolized using a nebulizer (Devilbiss, model 645, USA) at an airflow rate of 7 litre/minute. Sham-sensitized mice received 0.2 ml of saline containing containing only 2 mg Al(OH)$_3$ on days 0,7 and 14 followed by inhalation of aerosol of saline without ova for 5 consecutive days.

### Example 2-Treatment of mice with PBPB

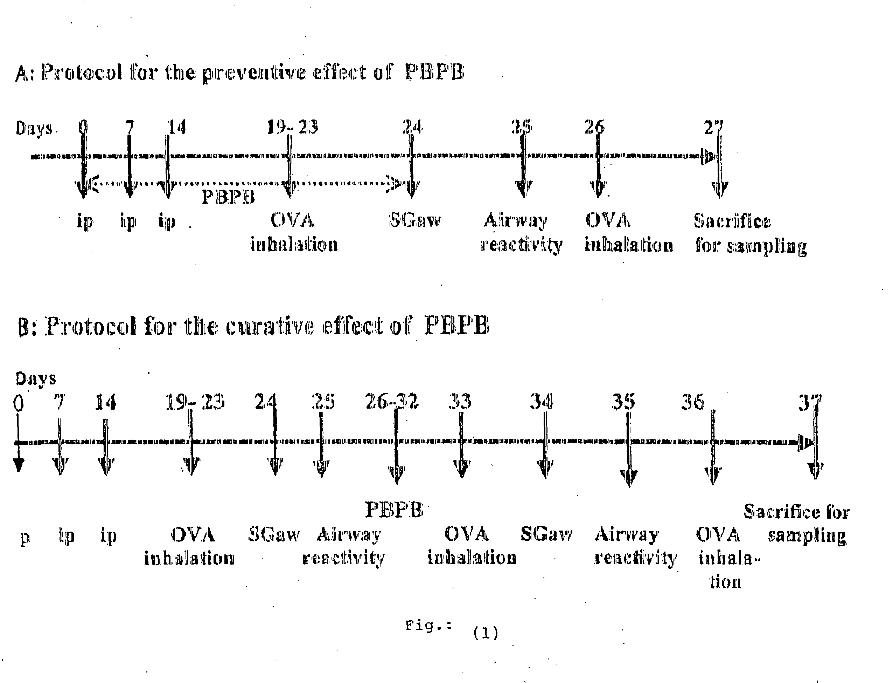

The compound PBPB (Sigma, USA) was dissolved in absolute alcohol (10 mg/ml). PBPB (20ul by volume) was given orally to each mouse. To evaluate the effect of PBPB on sensitization and the development of impaired airway sensitivity (preventive effect, Fig 1a), five groups (six mice in each) of mice were used. One group was sham-sensitized control and another group was kept as sensitized control. The remaining three groups were given orally three different concentrations of PBPB (0.1, and 10 mg/kg body weight) daily starting from first to the last day of the sensitization. The sham-sensitized and sensitized control mice were given only vehicle (20 ul alcohol) in a similar fashion. Initial measurements of specific airway conductance and airway reactivity of all the animals were earned out before starting the experiment. Ovalbumin induced airway constriction and airway reactivity were measured after sensitization protocol was over. The measurements were done as described below (Examples 3, 4 and 5).

To examine the therapeutic effect of PBPB on sensitized mice (therapeutic effect, Fig, 1b), mice were sensitized as before without PBPB treatment and airway constriction to ova and airway reactivity to methacholine were measured as described below. Animals showing at least 40% fall in specific airway conductance to ova aerosol challenge were selected for this study. The sensitized mice were randomly divided into 4 groups of 6 mice in each group. Three groups of mice was given daily three different concentrations of PBPB in 20 ul of alcohol adjusted to contain PBPB in a dose of 0.1 and 10 mg/kg body weight respectively for week. The fourth group was fed only 20 ul alcohol for one week to be used as sensitized control. Airway constriction and airway reactivity were again measured thereafter.

### Example 3 measurement of airways calibre:

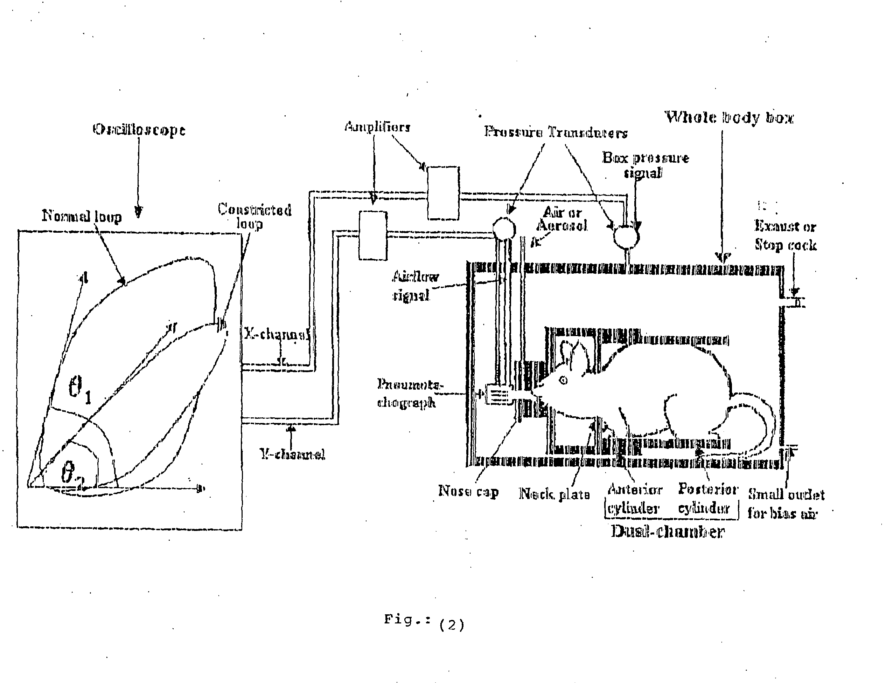

Airway calibre was measured in the term of specific airway conductance (SGaw). SGaw is a measure of the airway calibre and was estimated using a dual-chamber whole body plethysmograph(Agrawal, 1981), The &6cbembw whole body plethysmograph was designed in our laboratory to suit the size of mouse (Fig 2). The change in the box pressure in response to breathing of the housed mouse was determined with a transducer (validyne mp 45 + 2 cmh20) and carrier amplifier (validyne model CD 15 carrier demodulator) that were connected to the x-channel of an oscilloscope Tektronix, model 6116, USA). The pneumotachograph attached to the anterior chamber of the plethysmograph was used to detect the airflow at the nares of the mouse. This signal was amplified by mother set of the same type of transducer and amplifier with in turn were connected to the y-channel of the oscilloscope these two channels (x-y) of box pressure versus air flow were joined and displayed as a loop on the oscilloscope. The slope (tan 0) of the early inspiratory limb of x-y loop provided the data for computing SGaw. The SGaw is the ratio of air flow change to flow-related change in box pressure during the transition from expiration to inspiration of breading mice which comes after derivation as

$$SGaw = \tan\theta \times \frac{ml/sec/div \text{ on ordinate}}{ml/div \text{ on abscissa}} \times \frac{1}{\{pressure (barometer) - pressure (vapour)\}}$$

Animals were acclimatized in the body box in the beginning of the study and at the time of recording the loop, each individual mouse was housed for 10 minutes to set the basal loop correctly. Values of the slopes were recorded on getting at least three similar loops. Several loops were examined before selecting the similar three loops for recording the values. The above formula was used to calculate the values of SGaw (Agrawal, 1981).

**Example 4 - PBPB inhibits acute ova-induced airway constriction:**

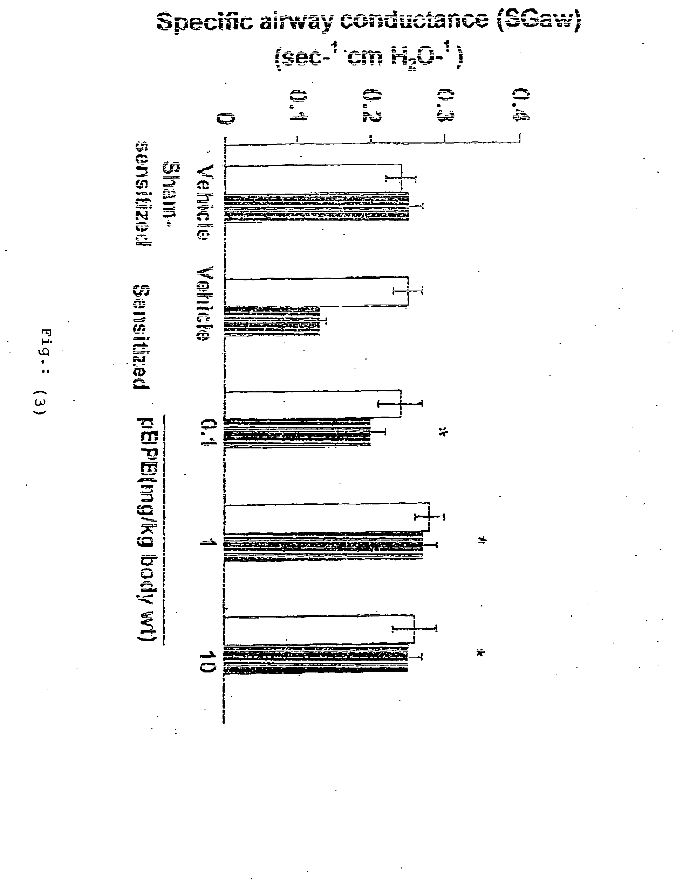

Airway constriction of mice was determined in terms of fall of SGaw due to ova aerosol challenge as described in *Example* 3. In order to study the preventive effect of PBPB on airway-constriction produced by sensitization, the mice were treated with PBPB 0.1, 1 and 10 mg/kg body weight) during sensitization period. The levels of SGaw were measured before and after ova aerosol challenge in all the groups (Fig. 3). The sham-sensitized mice did not show any significant decrease in their basal level of SGaw after ova challenge. The sensitized mice showed a fall of 48% from the basal value in SGaw level on challenge with ova. The oral treatment of animals with different doses of PBPB during sensitization period prevented the fall of SGaw levels in response to ova challenge in comparison to sensitized mice in a dose dependent manner. The mice treated with 0.1 mg PBPB/kg body weight showed a fall of SGaw level of only 17% while the animals which received mg PBPB/kg body weight had only 4% fall of SGaw from their basal levels. Further increase in the dose of PBPB to 10 mg/kg body weight did not show any additional effect (Fig. 3).

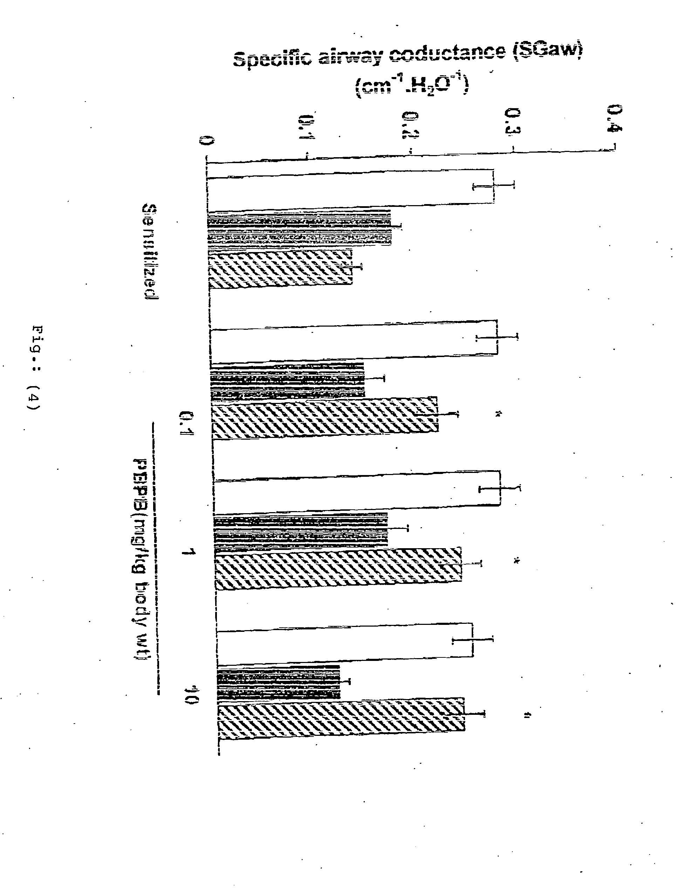

In order to study the therapeutic effect, PBPB (0.1, 1 and 10 mg/kg body weight) was administered orally for a period of one week to already sensitized animals. As shown in Fig. 4, it showed a reversal (curative effect) of SGaw in a dose dependent manner, a dose of mg/kg body weight recovered SGaw levels up to 86% of normal basal levels and the dose, 10 mg PBPB/kg body weight showed almost recovered (96%) to its basal SGaw levels (Fig. 4).

**Example 5: PBPB reduces airway reactivity to methacholine (MCh)**

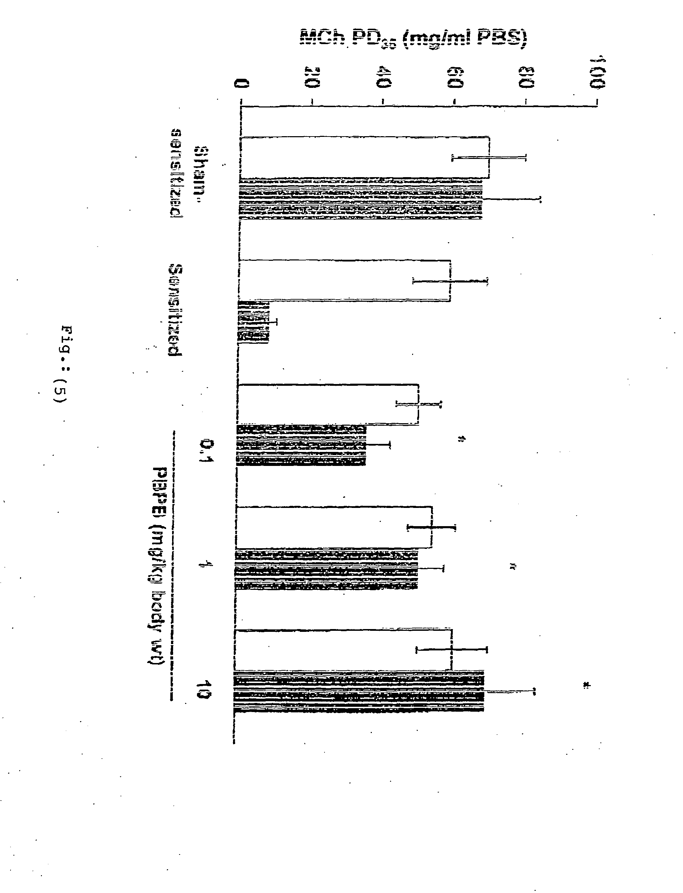

Airway reactivity to acetyl-beta-methacholine (Sigma, USA) was determined 24 hours after the last ova aerosol inhalation challenge. Aerosol of different concentration of methacholine (3.1, 6.25, 125, 50, 100 mg/ml) were given for 60 seconds. MCh $PD_{35}$ values were determined in sham-sensitized, sensitized and PBPB treated mice during sensitization and after as shown in the Fig. 5, there was no change in the MCh $PD_{35}$ values of sham-sensitized animals before and after challenge with ova, where as there was a significant fall in the MCh$PD_{35}$ value (86%) in sensitized mice 24 hrs after ova challenge as compared to its initial values (p < 0.005). a decrease in MCh $PD_{35}$ of airways of these sensitized mice clearly indicated their increased airway hyper reactivity to MCh as also observed in terms of fall in SGaw levels. The treatment of mice with PBPB during sensitization markedly attenuated MCh pd35 values towards basal level. The degree of attention of MCh $PD_{35}$ was dose dependent and a recovery of 93% of its initial levels was seen on treatment with mg/kg body weight.

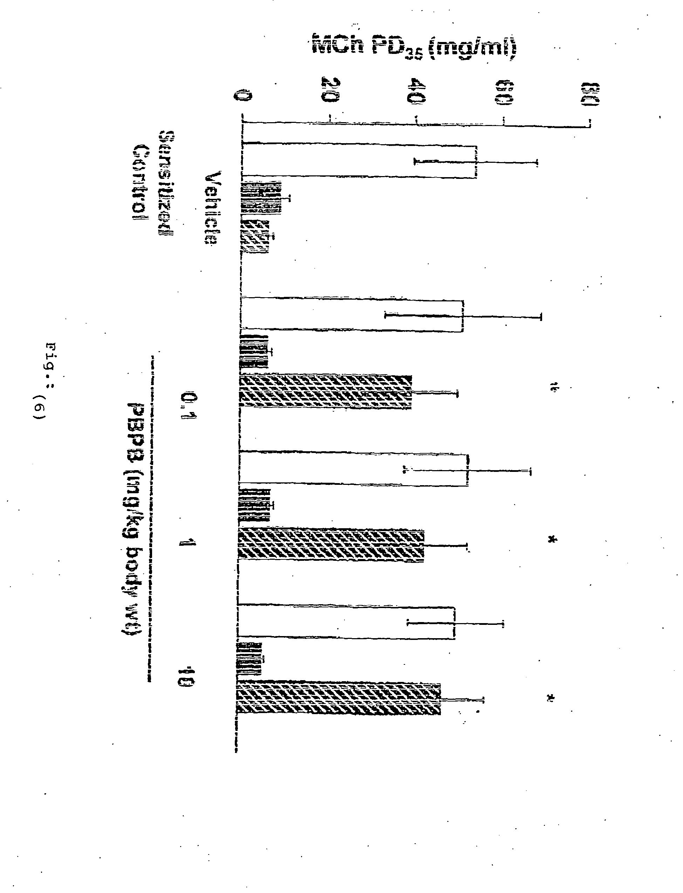

The dose 10 mg/kg body weight of PBPB showed still a better response, increasing the SGaw (115%) of its initial MCh pd3S values (Fig. 5). In addition, when the sensitized animals already having airway hyper reactivity (MCh $pd_{35}$ 5.5 $\pm$ 0.06 to 8.7$\pm$2.2) were treated orally with different concentrations of PBPB, their MCh $PD_{35}$ values were also recovered in a dose dependent manner (Fig. 6). The value were recovered by over 81% of the basal value at the dose 1 mg/kg body weight (Fig. 6) and the dose 10 mg/kg body weight showed a recovery of 94% of the basal MCh pd35 value. In sham-sensitized mice, aerosol challenge by different doses of methacholine did not demonstrate any change in MCh $pd_{35}$ value from its initial values after ova challenge

**Examples 6.PBPB treatment during and after sensitization reduces serum IgE levels.**

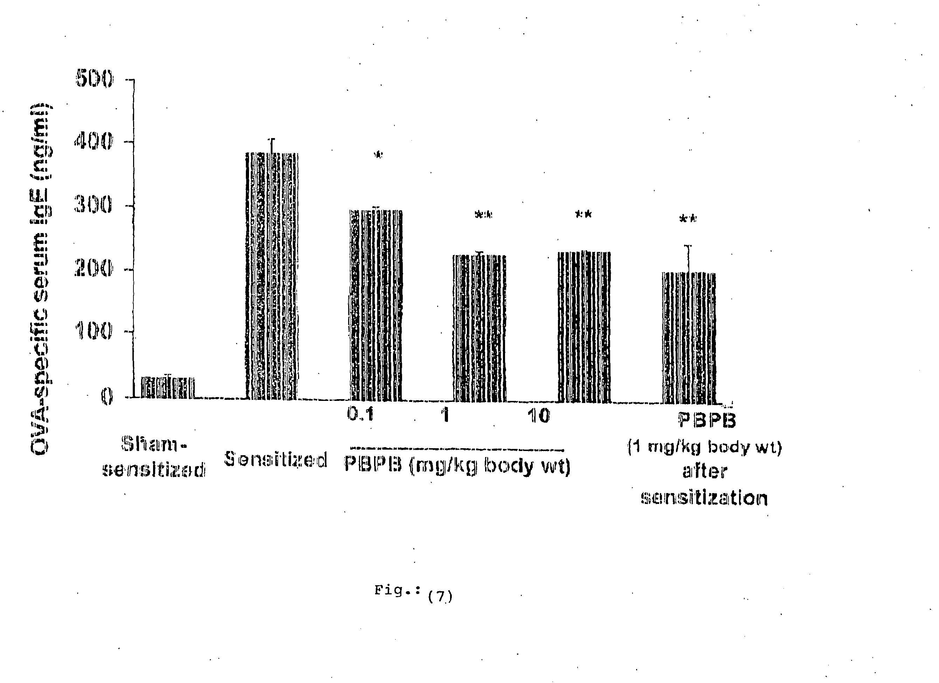

Serum IgE levels were measured in all the groups of mice by Elisa (Fig. 7). Ova-specific IgE levels were measured by enzyme linked immuno sorbent assay (Elisa). The IgE level increased markedly (384 $\pm$ 22.8 ng/ml) in the sensitized mice as compared to the sham-sensitized group (30 $\pm$ ng/ml) (Fig. 7). Interestingly, the oral administration of PHPB to the mice undergoing sensitization prevented the rise in serum IgE levels in a dose dependent manner. The mean serum IgE level of mice fed with PBPB (1 mg/kg body weight) was significantly lower (40 %) compared to those of sensitized animals. However, treatment with 10 mg of PBPB/ kg body weight did not show additional effect on IgE levels. Also, when PBPB (1 mg/kg body weight) was fed for one week to already sensitized mice, there was a significant (p <0.05) fall in the IgB levels in the serum (47%) as compared to those of the vehicle treated sensitized mice (Fig. 7).

**Example 7: PBPB Inhibits eosinophils in BAL fluid.**

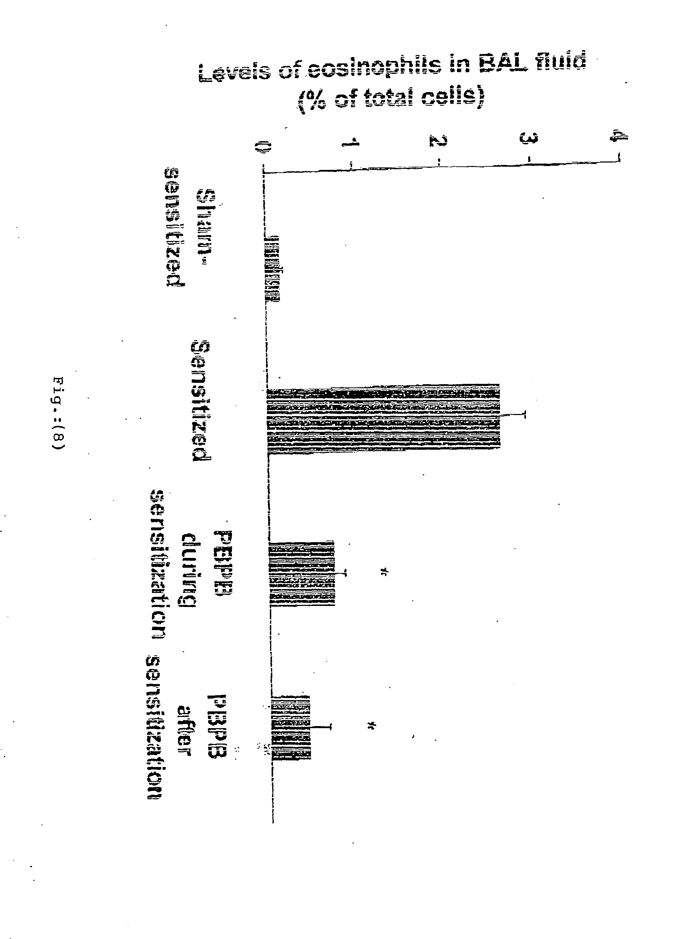

The levels of eosinophils in BAL fluid were detected by flowcytometry (facs Vantage, Becton Dickinson, USA) using the method described by Bedner et al (1999). The cells were gated on the basis of the size and fluorescence (FSC versus FL 1) and the percentage of the gated cells was determined by cell quest software. The data is represented as average values of six mice. As shown in Fig 8, the levels of eosinophils in BAL fluid were markedly elevated in ova sensitized mice as compared to those of sham-sensitized mice. The oral PBPB treatment (1 mg/kg body weight) during sensitization prevented the rise in the eosinophils levels. In already sensitized mice, treatment with PBPB (1 mg/kg body weight) also reduced the levels of eosinophils significantly ($p < 0.01$) as compared to those of the sensitized mice treated with only vehicle.

**Example 8 PBPB increases IFN-gamma and decreases IL-4 and IL-5 in BAL fluid.**

The cytokines IFN-gamma, IL-4 and IL-5 were measured in the BAL fluid by enzyme-linked ImmunoSorbent assay (Elisa) kits (BD pharmingen, USA) as per the manufacturer's protocol.

**Table-1**

| Treatment | Cytokines | | | |
|---|---|---|---|---|
| | IFN-$\gamma$ (pg/ml) | IL-4 (pg/ml) | IFN-$\gamma$:IL-4 | IL-5 (pg/ml) |
| Sham-sensitized | $495.0 \pm 152.5$ | $77.0 \pm 14.9$ | 6.4 | $104.8 \pm 15.2$ |
| Sensitized | $162.0 \pm 45.2$ | $143.0 \pm 3.0$ | 1.1 | $158.7 \pm 22.5$ |
| PBPB during Sensitization | $402 \pm 14.4$* | $91.0 \pm 13.2$* | 4.4 | $75.1 \pm 7.4$* |
| PBPB after Sensitization | $425.1 \pm 54.0$* | $42.4 \pm 4.9$* | 10.0 | $53.4 \pm 2.2$* |

The mice were treated with PBPB (1mg/kg body weight) during sensitization and after sensitization. The cytokines levels in BAL fluid were measured after sensitization and treatment with PBPB. Data are expressed as mean 1 SEM (n = 4 in each group). *Significantly different ($p < 0.05$) from the group of sensitized mice.

The results were given in pg/ml of each sample. The results (Table 1) showed that PBPB affects the level cytokines. The levels of IL-4 (143.0+3.0pg/ml) and IL-5 (158.7+22.5) were increased in sensitized mice as compared to those of sham-sensitized mice (77.0 + 14.9 pg/ml and 104.8 + 152 pg/ml respectively). The PBPB treatment during sensitization protocol or after sensitization were reduced significantly (p. 0.05) as compared to sensitized mice. On the other hand PBPB attenuated the levels of IFN-y to towards the levels of sham-sensitized mice. In both the groups of mice treated with PBPB during sensitization and after sensitization, there was an increase in the ratio af IFN-y to IL-4 (4.4 and 10 respectively) as compared to those of sensitized control mice (1.1).

**The main advantages of the present invention:**

1. This is the first demonstration that PBPB inhibits the characteristic features of asthma produced in an animal model and can be used for development of effective drugs for asthma therapy.

2. PBPB, being a non-steroidal compound, may have lesser side effects than the existing therapeutic steroids.

3. This compound is inexpensive and readily available.

4. The use of PBPB may not be restricted only to anti-asthmatic agent, but to other inflammatory conditions where elevations of IgE, reduction in IL-4, IL-5 and eosinophils levels play significant roles.

**Claims**

1. Use of parabromophenacyl bromide (PBPB) for the manufacture of a medicament for preventing and/or treating asthma.

2. Use as claimed in claim 1, wherein the medicament is for an animal or a human subject.

3. Use as claimed in claim 1 or claim 2, wherein the concentration of PBPB is ranging between 0.1 to 10 mg/kg body weight of the subject.

4. Use as claimed in claim 1 or claim 2, wherein the concentration of PBPB is 1 mg/kg body weight of the subject.

5. Use as claimed in any one of the preceding claims, wherein the PBPB is to be administered for one week.

6. Use as claimed in any one of the preceding claims, wherein the PBPB is to be administered through a route selected from a group comprising the intra-peritoneum and the oral routes.

7. Use of parabromophenacyl bromide (PBPB) for the manufacture of a medicament for modulating levels of biomolecules to help prevent and/or treat asthma.

8. Use as claimed in claim 7, wherein the PBPB helps maintains IFN-gamma levels.

9. Use as claimed in claim 7, wherein the PBPB helps attenuate IL-4, IL-5 and IL-13 levels.

10. Use as claimed in claim 7, wherein the PBPB prevents an increase in IL-4, IL-5 and IL-13 levels.

11. Use as claimed in claim 7, wherein the PBPB helps attenuate eosinophils levels.

12. Use as claimed in claim 7, wherein the PBPB prevents an increase in eosinophils levels.

13. Use as claimed in claim 7, wherein the PBPB helps attenuate IgE levels by 50%.

14. Use as claimed in claim 7, wherein the PBPB prevents an increase in IgE levels.

15. Use as claimed in claim 7, wherein the PBPB helps prevent airway constriction (SGaw).

16. Use as claimed in claim 7, wherein the PBPB helps recover up to 96% of basal level airway constriction (SGaw).

17. Use as claimed in claim 7, wherein the PBPB helps prevent airway reactivity.

**Patentansprüche**

1. Verwendung von Parabromphenacylbromid (PBPB) zur Herstellung eines Medikaments für die Vorbeugung und/oder Behandlung von Asthma.

2. Verwendung nach Anspruch 1, wobei das Medikament für ein Tier oder einen menschlichen Patienten ist.

3. Verwendung nach Anspruch 1 oder Anspruch 2, wobei die Konzentration von PBPB zwischen 0,1 und 10 mg/kg Körpergewicht des Patienten liegt.

4. Verwendung nach Anspruch 1 oder Anspruch 2, wobei die Konzentration von PBPB 1 mg/kg Körpergewicht des Patienten beträgt.

5. Verwendung nach einem der vorhergehenden Ansprüche, wobei PBPB für eine Woche verabreicht werden soll.

6. Verwendung nach einem der vorhergehenden Ansprüche, wobei PBPB auf einem Weg verabreicht werden soll, der aus einer Gruppe ausgewählt ist, die den intraperitonealen und oralen Weg umfaßt.

**7.** Verwendung von Parabromphenacylbromid (PBPB) zur Herstellung eines Medikaments für die Modulation von Niveaus an Biomolekülen, um die Vorbeugung und/oder Behandlung von Asthma zu unterstützen.

**8.** Verwendung nach Anspruch 7, wobei PBPB die Aufrechterhaltung von IFN-gamma-Niveaus unterstützt.

**9.** Verwendung nach Anspruch 7, wobei PBPB die Verringerung von IL-4-, IL-5- und IL-13-Niveaus unterstützt.

**10.** Verwendung nach Anspruch 7, wobei PBPB eine Erhöhung von IL-4-, IL-5- und IL-13-Niveaus verhindert.

**11.** Verwendung nach Anspruch 7, wobei PBPB die Verringerung von Eosinophilniveaus unterstützt.

**12.** Verwendung nach Anspruch 7, wobei PBPB eine Erhöhung von Eosinophilniveaus verhindert.

**13.** Verwendung nach Anspruch 7, wobei PBPB die Verringerung von IgE-Niveans um 50 % unterstützt.

**14.** Verwendung nach Anspruch 7, wobei PBPB eine Erhöhung von 1gE-Niveaus verhindert.

**15.** Verwendung nach Anspruch 7, wobei PBPB die Vorbeugung von Atemwegskonstriktion (SGaw) unterstützt.

**16.** Verwendung nach Anspruch 7, wobei PBPB die Wiederherstellung von bis zu 96 % der Grundniveau-Atemwegskonstriktion (SGaw) unterstützt.

**17.** Verwendung nach Anspruch 7, wobei PBPB die Verhinderung der Aternwegsreaktivität unterstützt.

**Revendications**

**1.** Utilisation de bromure de parabromophénacyle (pBPB) pour la fabrication d'un médicament destiné à la prévention et/ou au traitement de l'asthme.

**2.** Utilisation selon la revendication 1, où le médicament est destiné à un sujet animal ou humain.

**3.** Utilisation selon la revendication 1 ou la revendication 2, où la concentration de pBPB se situe dans la plage de 0,1 mg/kg à 10 mg/kg de poids corporel du sujet.

**4.** Utilisation selon la revendication 1 ou la revendication 2, où la concentration de pBPB est de 1 mg/kg de poids corporel du sujet.

**5.** Utilisation selon l'une quelconque des revendications précédentes, où le pBPB doit être administré pendant une semaine.

**6.** Utilisation selon l'une quelconque des revendications précédentes, où le pBPB doit être administré par l'intermédiaire d'une voie choisie dans un groupe comprenant les administrations par voie intrapéritonéale et par voie orale.

**7.** Utilisation de bromure de parabromophénacyle (pBPB) pour la fabrication d'un médicament destiné à la modulation des taux de biomolécules pour aider à prévenir et/ou traiter l'asthme.

**8.** Utilisation selon la revendication 7, où le pBPB aide à maintenir les taux d'IFN-gamma.

**9.** Utilisation selon la revendication 7, où le pBPB aide à atténuer les taux d'IL-4, d'IL-5 et d'IL-13.

**10.** Utilisation selon la revendication 7, où le pBPB empêche une augmentation des taux d'IL-4, d'IL-5 et d'IL-13.

**11.** Utilisation selon la revendication 7, où le pBPB aide à atténuer les taux d'éosinophiles.

**12.** Utilisation selon la revendication 7, où le pBPB empêche une augmentation des taux d'éosinophiles.

**13.** Utilisation selon la revendication 7, où le pBPB aide à atténuer les taux d'IgE de 50 %.

**14.** Utilisation selon la revendication 7, où le pBPB empêche une augmentation des taux d'IgE.

**15.** Utilisation selon la revendication 7, où le pBPB aide à prévenir une constriction des voies respiratoires (sGaw).

**16.** Utilisation selon la revendication 7, où le pBPB aide à récupérer jusqu'à 96 % du taux de base de constriction des voies respiratoires (sGaw).

**17.** Utilisation selon la revendication 7, où le pBPB aide à empêcher la réactivité des voies respiratoires.

EP 1 569 631 B1

**A: Protocol for the preventive effect of PBPB**

Days    0    7    14    19-23    24    25    26    27

PBPB

ip    ip    ip    OVA inhalation    SGaw    Airway reactivity    OVA inhalation    Sacrifice for sampling

**B: Protocol for the curative effect of PBPB**

Days
0    7    14    19-23    24    25    26-32    33    34    35    36    37

PBPB

p    ip    ip    OVA inhalation    SGaw    Airway reactivity    OVA inhalation    SGaw    Airway reactivity    OVA inhalation    Sacrifice for sampling

Fig.: (1)

Fig.: (2)

Specific airway conductance (SGaw)
(sec-¹·cm H₂O-¹)

Fig.: (3)

Fig.: (4)

Fig.: (5)

EP 1 569 631 B1

Fig.: (6)

16

Fig.: (7)

Levels of eosinophils in BAL fluid
(% of total cells)

Fig.:(8)